(19)

**Europäisches Patentamt**
**European Patent Office**
**Office européen des brevets**

(11) **EP 4 365 219 A1**

(12) **EUROPEAN PATENT APPLICATION**
published in accordance with Art. 153(4) EPC

(43) Date of publication:
**08.05.2024 Bulletin 2024/19**

(21) Application number: **22832557.7**

(22) Date of filing: **31.03.2022**

(51) International Patent Classification (IPC):
**C08G 73/10** *(2006.01)*   **C08J 3/12** *(2006.01)*
**C08J 3/24** *(2006.01)*

(52) Cooperative Patent Classification (CPC):
**C08G 73/10; C08J 3/12; C08J 3/24**

(86) International application number:
**PCT/JP2022/016512**

(87) International publication number:
**WO 2023/276396 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2021   JP 2021107638**

(71) Applicants:
• **DIC Corporation**
  **Tokyo 174-8520 (JP)**
• **Green Earth Institute Co., Ltd.**
  **Tokyo 160-0022 (JP)**

(72) Inventors:
• **KONDO, Akihiro**
  **Sakura-shi, Chiba 285-8668 (JP)**
• **OKA, Yasutaka**
  **Sakura-shi, Chiba 285-8668 (JP)**
• **SASAMOTO, Akane**
  **Sakura-shi, Chiba 285-8668 (JP)**
• **ARAI, Tatsuhiko**
  **Sakura-shi, Chiba 285-8668 (JP)**
• **NAKAYASHIKI, Toru**
  **Kisarazu-shi, Chiba 292-0818 (JP)**
• **MIURA, Satomi**
  **Kisarazu-shi, Chiba 292-0818 (JP)**

(74) Representative: **Kraus & Lederer PartGmbB**
  **Thomas-Wimmer-Ring 15**
  **80539 München (DE)**

(54) **ASPARTIC ACID COMPOSITION, POLYSUCCINIMIDE COMPOSITION, POLYASPARTIC ACID COMPOSITION, AND CROSS-LINKED POLYASPARTIC ACID COMPOSITION**

(57)     Provided is an aspartic acid composition containing aspartic acid and an impurity, the impurity containing at least one selected from the group consisting of an amino acid other than aspartic acid, an organic acid, and salts of the amino acid other than aspartic acid and the organic acid, a content of the impurity being 0.02% by mass or more and 1.1% by mass or less. Also provided is a method for producing polysuccinimide, the method including performing a polymerization reaction of the aspartic acid using the aspartic acid composition. Also provided is a polysuccinimide composition produced by the method for producing polysuccinimide.

EP 4 365 219 A1

**Description**

Technical Field

The present invention relates to an aspartic acid composition, a polyaspartic acid composition, a cross-linked polyaspartic acid composition, a surface-modified cross-linked polyaspartic acid composition, and methods for producing them. The present invention also relates to a polysuccinimide composition, a cross-linked polysuccinimide compound, and methods for producing them. The present invention further relates to a cross-linked polyaspartic acid-containing product and a method for producing a cross-linked polyaspartic acid-containing product.

The present application claims priority based on Japanese Patent Application No. 2021-107638 filed in Japan on June 29, 2021, the contents of which are hereby incorporated herein by reference.

Background Art

Polyaspartic acid is a polycarboxylic acid having biodegradability and resembles polyacrylic acid in chemical structure. For this reason, polyaspartic acid is expected as a biodegradable material with high water retainability to replace polyacrylic acid. As a method for obtaining polyaspartic acid, known is a method of obtaining polysuccinimide by a dehydration-condensation reaction of aspartic acid and then performing alkaline hydrolysis of the polysuccinimide (PTL 1).

Aspartic acid, which is a raw material of polysuccinimide, is industrially synthesized using aspartase with petroleum-derived fumaric acid and ammonia as raw materials. However, due to problems such as the depletion of petroleum resources, a method of producing aspartic acid by a bioprocess using microorganisms is being considered (PTL 2) .

Citation List

Patent Literature

PTL 1: Japanese Unexamined Patent Application Publication No. H09-176312
PTL 2: WO 2020/208842

Summary of Invention

Technical Problem

The inventors of the present invention recognized that in the step of obtaining polyaspartic acid from aspartic acid, the polymerization reaction rate of polysuccinimide, which is an intermediate, was low. To make the step of obtaining polyaspartic acid from aspartic acid efficient, it is desirable to increase the polymerization reaction rate of polysuccinimide, which is an intermediate, and make it a polymer with a high molecular weight. It is also desirable to efficiently obtain cross-linked polyaspartic acid with high water retainability using aspartic acid.

Given these circumstances, an object of the present invention is to provide an aspartic acid composition capable of being used for efficient production of a cross-linked polyaspartic acid composition. Another object of the present invention is to provide a polysuccinimide composition containing polysuccinimide with a high molecular weight, a cross-linked polyaspartic acid composition with high water retainability, and the like obtained from the aspartic acid composition.

Solution to Problem

The present invention includes the following aspects.

[1] An aspartic acid composition containing aspartic acid and an impurity, the impurity containing at least one selected from the group consisting of an amino acid other than aspartic acid, an organic acid, and salts of the amino acid other than aspartic acid and the organic acid, a content of the impurity being 0.02% by mass or more and 1.1% by mass or less.
[2] The aspartic acid composition according to [1], in which the impurity contains the amino acid other than aspartic acid.
[3] The aspartic acid composition according to [1] or [2], in which the aspartic acid is produced by microorganisms.
[4] A method for producing polysuccinimide, the method including performing a polymerization reaction of the aspartic acid using the aspartic acid composition according to any one of [1] to [3].

[5] The method for producing polysuccinimide according to [4], in which the polymerization reaction is performed within a kneading apparatus.

[6] A polysuccinimide composition containing polysuccinimide and an impurity, the impurity containing at least one selected from the group consisting of an amino acid other than aspartic acid, an organic acid, and salts of the amino acid other than aspartic acid and the organic acid, a content of the impurity being 0.001% by mass or more and 1.1% by mass or less.

[7] The polysuccinimide composition according to [6], in which the impurity contains the amino acid other than aspartic acid.

[8] A polysuccinimide composition, being a polymerization reaction product of the aspartic acid composition according to any one of [1] to [3].

[9] A polyaspartic acid composition, being a hydrolyzed product of the polysuccinimide composition according to any one of [6] to [8].

[10] A polyaspartic acid composition containing polyaspartic acid and an impurity, the impurity containing at least one selected from the group consisting of an amino acid other than aspartic acid, an organic acid, and salts of the amino acid other than aspartic acid and the organic acid, a content of the impurity being 0.001% by mass or more and 1.1% by mass or less.

[11] The polyaspartic acid composition according to [10], in which the impurity contains the amino acid other than aspartic acid or a salt of the amino acid other than aspartic acid.

[12] A cross-linked polyaspartic acid composition, being a cross-linking reaction product of the polyaspartic acid composition according to any one of [9] to [11].

[13] A cross-linked polysuccinimide composition, being a cross-linking reaction product of the polysuccinimide composition according to any one of [6] to [8].

[14] A cross-linked polyaspartic acid composition, being a hydrolyzed product of the cross-linked polysuccinimide composition according to [13].

[15] A surface-modified cross-linked polyaspartic acid composition containing particles of the cross-linked polyaspartic acid composition according to [12] or [14], surfaces of the particles being modified with inorganic particles.

[16] A cross-linked polyaspartic acid-containing product containing the cross-linked polyaspartic acid composition according to [12] or [14] or the surface-modified cross-linked polyaspartic acid composition according to [15].

[17] The cross-linked polyaspartic acid-containing product according to [15], in which the cross-linked polyaspartic acid-containing product is a surfactant, a viscosity-increasing agent, a viscosity-reducing agent, a swelling agent, a water absorbent, a gelator, a binder, a film-forming agent, a flocculant, an adhesive, a surface modifier, or a sustained-release agent.

[18] A method for producing a polyaspartic acid composition, the method including a step of performing a polymerization reaction of the aspartic acid using the aspartic acid composition according to any one of [1] to [3] to obtain a polysuccinimide composition containing polysuccinimide and a step of performing a hydrolysis reaction of the polysuccinimide using the polysuccinimide composition to obtain a polyaspartic acid composition containing polyaspartic acid.

[19] A method for producing a cross-linked polyaspartic acid composition, the method including a step of obtaining a polyaspartic acid composition by the method for producing a polyaspartic acid composition according to [18] and a step of performing a cross-linking reaction of the polyaspartic acid using the polyaspartic acid composition to obtain a cross-linked polyaspartic acid composition containing cross-linked polyaspartic acid.

[20] A method for producing a cross-linked polyaspartic acid composition, the method including a step of performing a polymerization reaction of the aspartic acid using the aspartic acid composition according to any one of [1] to [3] to obtain a polysuccinimide composition containing polysuccinimide, a step of performing a cross-linking reaction of the polysuccinimide using the polysuccinimide composition to obtain a cross-linked polysuccinimide composition containing cross-linked polysuccinimide, and a step of performing a hydrolysis reaction of the cross-linked polysuccinimide using the cross-linked polysuccinimide composition to obtain a cross-linked polyaspartic acid composition containing cross-linked polyaspartic acid.

[21] A method for producing a cross-linked polyaspartic acid-containing product, the method including a step of obtaining a cross-linked polyaspartic acid composition by the method for producing a cross-linked polyaspartic acid composition according to [19] or [20] and a step of producing a cross-linked polyaspartic acid-containing product using the cross-linked polyaspartic acid composition.

[22] The method for producing a cross-linked polyaspartic acid-containing product according to [21], in which the cross-linked polyaspartic acid-containing product is a surfactant, a viscosity-increasing agent, a viscosity-reducing agent, a swelling agent, a water absorbent, a gelator, a binder, a film-forming agent, a flocculant, an adhesive, a surface modifier, or a sustained-release agent. Advantageous Effects of Invention

According to the present invention, the aspartic acid composition contains the impurity such as the amino acid or the organic acid in a certain amount, and thereby a polysuccinimide compound containing polysuccinimide with a high molecular weight can be obtained by performing a polymerization reaction using the aspartic acid composition. In addition, a cross-linked polyaspartic acid composition with high water retainability can be obtained from the polysuccinimide composition.

Brief Description of Drawings

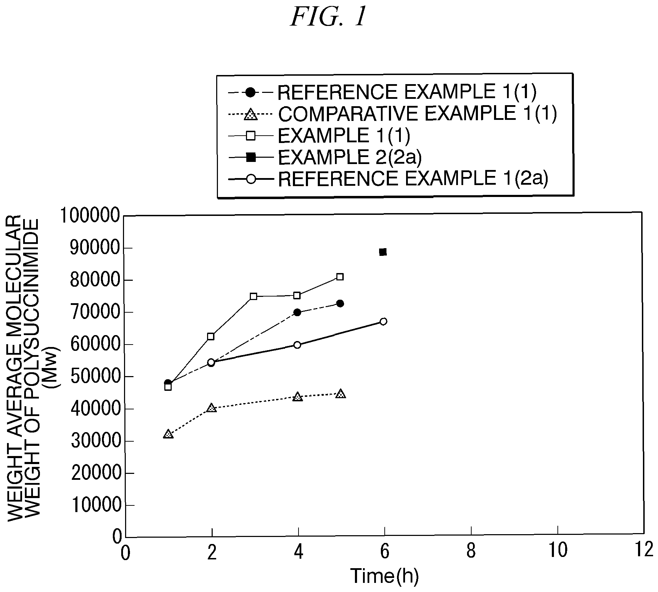

FIG. 1 illustrates a result of the weight average molecular weight (Mw) of polysuccinimide produced by a polymerization reaction of an aspartic acid composition measured over time.

Description of Embodiments

The following describes the present invention in more detail. The present invention is not limited only to the embodiments described below. In the present specification, "to" means a value before the description of "to" or more and a value after the description of "to" or less.

[Aspartic Acid Composition]

In one aspect, the present invention provides an aspartic acid composition for producing polysuccinimide. The aspartic acid composition contains aspartic acid and an impurity. The impurity contains at least one selected from the group consisting of an amino acid other than aspartic acid, an organic acid, and salts thereof. The content of the impurity in the aspartic acid composition is 0.02% by mass or more and 1.1% by mass or less.

The "aspartic acid composition" refers to a composition containing aspartic acid as a main component. The aspartic acid composition is used for production of polysuccinimide. The aspartic acid composition contains aspartic acid and an impurity.

<Impurity>

The "impurity" of the aspartic acid composition refers to a component other than aspartic acid contained in the aspartic acid composition. In one embodiment, the impurity contains at least one selected from the group consisting of an amino acid other than aspartic acid, an organic acid, and salts thereof.

The "amino acid" is an organic substance having both an amino group and a carboxy group. The amino acid contained as the impurity in the aspartic acid composition is an amino acid other than aspartic acid. The amino acid contained as the impurity may be any of an α-amino acid, a β-amino acid, a γ-amino acid, and a 5-amino acid. The amino acid may be an L-body, a D-body, or a mixture of these. In one embodiment, the amino acid is an L-body. The impurity preferably contains the amino acid other than aspartic acid.

Examples of the amino acid as the impurity include alanine, arginine, asparagine, cysteine, glutamine, glutamic acid, glycine, histidine, isoleucine, leucine, lysine, methionine, phenylalanine, proline, serine, threonine, tryptophan, tyrosine, and valine. Among them, the impurity preferably contains at least one selected from the group consisting of glutamic acid, alanine, and valine and more preferably contains glutamic acid and/or valine.

The content of the amino acid as the impurity is preferably 0 part by mass or more and 1.1 parts by mass or less with respect to 100 parts by mass of aspartic acid, in which the lower limit may be 0.001 part by mass or more, 0.005 part by mass or more, 0.010 part by mass or more, 0.015 part by mass or more, 0.02 part by mass or more, 0.03 part by mass or more, 0.05 part by mass or more, 0.07 part by mass or more, 0.09 part by mass or more, 0.10 part by mass or more, 0.15 part by mass or more, 0.18 part by mass or more, 0.20 part by mass or more, 0.25 part by mass or more, and the upper limit may be 1.1 parts by mass or less, 1.0 part by mass or less, 0.98 part by mass or less, 0.95 part by mass or less, 0.90 part by mass or less, 0.80 part by mass or less, 0.75 part by mass or less, 0.70 part by mass or less, 0.50 part by mass or less, or 0.30 part by mass or less. These values of the upper limit and the lower limit are used in any combination.

The content of the amino acid as the impurity may be 0 part by mass or more and 1.1 parts by mass or less, 0.02 part by mass or more and 1.1 parts by mass or less, 0.02 part by mass or more and 1.0 part by mass or less, 0.001 part by mass or more and 0.98 part by mass or less, 0.005 part by mass or more and 0.95 part by mass or less, 0.010 part by mass or more and 0.90 part by mass or less, 0.020 part by mass or more and 0.80 part by mass or less, 0.05 part by mass or more and 0.70 part by mass or less, 0.05 part by mass or more and 0.50 part by mass or less, or 0.05 part by mass or more and 0.30 part by mass or less with respect to 100 parts by mass of aspartic acid.

The amino acid as the impurity may be in salt form. Examples of the salt of the amino acid include salts with alkali metals (sodium, potassium, lithium, and the like) and salts with alkaline earth metals (calcium, magnesium, and the like). The content ranges exemplified for the amino acid as the impurity also apply when the amino acid as the impurity is in salt form. When both the amino acid and the salt of the amino acid are contained as the impurity, the content ranges exemplified above apply to the total content of both.

The "organic acid" is an organic substance showing acidity. In one embodiment, the organic acid is a carboxylic acid. When the "organic acid" is used in the present specification, the amino acid is excluded.

Examples of the organic acid as the impurity include pyruvic acid, maleic acid, fumaric acid, malic acid, acetic acid, and succinic acid.

The content of the organic acid as the impurity is preferably 0 part by mass or more and 1.1 parts by mass or less with respect to 100 parts by mass of aspartic acid, in which the lower limit may be 0.02 part by mass or more, 0.03 part by mass or more, 0.05 part by mass or more, 0.07 part by mass or more, 0.09 part by mass or more, 0.10 part by mass or more, 0.15 part by mass or more, 0.18 part by mass or more, 0.20 part by mass or more, or 0.25 part by mass or more, and the upper limit may be 1.1 parts by mass or less, 1.0 part by mass or less, 0.98 part by mass or less, 0.95 part by mass or less, 0.90 part by mass or less, 0.80 part by mass or less, 0.75 part by mass or less, 0.70 part by mass or less, 0.50 part by mass or less, or 0.30 part by mass or less. These values of the upper limit and the lower limit are used in any combination.

The content of the organic acid as the impurity may be 0 part by mass or more and 1.1 parts by mass or less, 0.001 part by mass or more and 1.1 parts by mass or less, 0.02 part by mass or more and 1.1 parts by mass or less, 0.02 part by mass or more and 1.0 part by mass or less, 0.001 part by mass or more and 0.98 part by mass or less, 0.005 part by mass or more and 0.95 part by mass or less, 0.010 part by mass or more and 0.90 part by mass or less, 0.020 part by mass or more and 0.80 part by mass or less, 0.05 part by mass or more and 0.70 part by mass or less, 0.05 part by mass or more and 0.50 part by mass or less, or 0.05 part by mass or more and 0.30 part by mass or less with respect to 100 parts by mass of aspartic acid.

The organic acid as the impurity may be in salt form. Examples of the salt of the organic acid include salts with alkali metals (sodium, potassium, lithium, and the like) and salts with alkaline earth metals (calcium, magnesium, and the like). The content ranges exemplified for the organic acid as the impurity also apply when the organic acid as the impurity is in salt form. When both the organic acid and the salt of the organic acid are contained as the impurity, the content ranges exemplified above apply to the total content of both.

The aspartic acid composition contains the impurity in an amount of 0.02% by mass or more 1.1% by mass or less with respect to the total mass of all the components of the aspartic acid composition. The content of the impurity is preferably 0.021% by mass or more and 1.08% by mass or less with respect to the total mass of all the components of the aspartic acid composition, in which the lower limit may be 0.021% by mass or more, 0.025% by mass or more, 0.03% by mass or more, 0.035% by mass or more, 0.04% by mass or more, 0.045% by mass or more, 0.05% by mass or more, 0.07% by mass or more, 0.09% by mass or more, 0.10% by mass or more, 0.15% by mass or more, 0.18% by mass or more, 0.20% by mass or more, or 0.25% by mass or more, and the upper limit may be 1.05% by mass or less, 1.0% by mass or less, 0.98% by mass or less, 0.95% by mass or less, 0.90% by mass or less, 0.80% by mass or less, 0.75% by mass or less, 0.70% by mass or less, 0.50% by mass or less, or 0.30% by mass or less. These values of the upper limit and the lower limit are used in any combination.

The content of the impurity may be 0.021% by mass or more and 1.1% by mass or less, 0.025% by mass or more and 1.1% by mass or less, 0.02% by mass or more and 1.0% by mass or less, 0.02% by mass or more and 0.98% by mass or less, 0.02% by mass or more and 0.95% by mass or less, 0.02% by mass or more and 0.90% by mass or less, 0.020% by mass or more and 0.80% by mass or less, 0.02% by mass or more and 0.70% by mass or less, 0.05% by mass or more and 0.50% by mass or less, or 0.05% by mass or more and 0.30% by mass or less with respect to the total mass of all the components of the aspartic acid composition.

The content of the impurity being within the above range promotes a polymerization reaction of aspartic acid. In addition, polysuccinimide with a high molecular weight can be obtained.

The impurity may contain components other than the amino acid other than aspartic acid, the organic acid, and salts thereof. Examples of the components include sulfates.

<Aspartic Acid>

Aspartic acid may be an L-body, a D-body, or a mixture of these. In one embodiment, aspartic acid is an L-body. Aspartic acid may be produced by microorganisms or produced without using microorganisms. Examples of the method for producing aspartic acid without using microorganisms include a method of reacting fumaric acid and ammonia in the presence of an enzyme such as aspartase.

The content of aspartic acid in the aspartic acid composition is 98.9% by mass or more and 99.98% by mass or less with respect to the total mass of all the components of the aspartic acid composition. The content of aspartic acid may be 98.95% by mass or more and 99.97% by mass or less, 99.00% by mass or more and 99.96% by mass or less, 99.02% by mass or more and 99.95% by mass or less, or 99.05% by mass or more and 99.93% by mass or less with respect to the total mass of all the components of the aspartic acid composition. These values of the upper limit and the lower limit are used in any combination.

<Method for Producing Aspartic Acid Composition>

The aspartic acid composition can be produced by known methods. Examples of the method for producing the aspartic acid composition include a method of reacting fumaric acid and ammonia in the presence of an enzyme such as aspartase and a method of utilizing microorganisms having aspartic acid producing ability such as bacteria of the genus Corynebacterium.

(Method using Microorganisms)

In the present specification, it is sufficient to interpret "microorganism" literally, and more specifically, "microorganism" may be prokaryotes such as archaea, cyanobacteria, and bacteria or eukaryotes such as fungi. The "microorganisms" in the present embodiment are preferably fungi or bacteria and more preferably bacteria.

Examples of the fungi include yeasts of the genus Saccharomyces (for example, Saccharomyces cerevisiae), yeasts of the genus Schizosaccharomyces (for example, Schizosaccharomyces pombe), yeasts of the genus Pichia (for example, Pichia pastoris), yeasts of the genus Kluyveromyces (Kluyveromyces lactis), Hansenula polymorpha, yeasts of the genus Yarrowia (for example, Yarrowia lipolytica), fungi of the genus Cryptococcus (for example, Cryptococcus sp. S-2), fungi of the genus Aspergillus (for example, Aspergillus oryzae), and fungi of the genus Pseudozyma (for example, Pseudozyma antarctica). Saccharomyces cerevisiae, Schizosaccharomyces pombe, Pichia pastoris, and the like can be conveniently utilized in the present invention because their genetic recombination techniques and heterologous protein expression systems are well established.

Examples of the bacteria include bacteria of the genus Escherichia (for example, Escherichia coli), bacteria of the genus Bacillus (for example, Bacillus subtilis), bacteria of the genus Lactobacillus (for example, Lactobacillus acidophilus), bacteria of the genus Clostridium (for example, Clostridium thermocellum and Clostridium acetobutylicum), bacteria of the genus Rhodopseudomonas (for example, Rhodopseudomonas palustris), bacteria of the genus Rhodobacter (Rhodobacter capsulatus), and bacteria belonging to coryneform bacteria described in detail below. The "microorganisms" in the present specification are preferably bacteria of the genus Escherichia or coryneform bacteria, for which genetic recombination techniques and protein expression systems have already been established and which enables substance production under reducing conditions, in which cells do not substantially proliferate, more preferably Escherichia coli or coryneform bacteria, and most preferably bacteria of the genus Corynebacterium.

The "coryneform bacteria" refers to a group of microorganisms defined in Bargeys Manual of Determinative Bacteriology, Vol. 8, p. 599, 1974.

More specifically, examples of coryneform bacteria include bacteria of the genus Corynebacterium, bacteria of the genus Brevibacterium, bacteria of the genus Arthrobacter, bacteria of the genus Mycobacterium, bacteria of the genus Micrococcus, and bacteria of the genus Microbacterium.

Examples of the bacteria of the genus Corynebacterium include Corynebacterium glutamicum.

When the microorganisms are utilized, the microorganisms may be cultured using a culture medium normally used for culture of heterotrophic bacteria. Examples of the culture medium include culture media containing carbon sources, nitrogen sources, phosphorus sources, trace elements, vitamins, or the like. Examples of the carbon sources include sugars (glucose, sucrose, lactose, fructose, maltose, molasses, starch, and the like), fatty acids (palmitic acid, stearic acid, linoleic acid, and the like), alcohols (glycerol, ethanol, and the like), and organic acids (acetic acid and the like). Examples of the nitrogen sources include nitrogen-containing organic compounds (peptone, yeast extracts, meat extracts, urea, amino acids, and the like), inorganic ammonium salts, and inorganic nitrates. Examples of the phosphorus sources include potassium dihydrogen phosphate and dipotassium hydrogen phosphate. Examples of the trace elements include magnesium, iron, and zinc. Examples of the vitamins include biotin and thiamine.

The culture conditions can be selected as appropriate in accordance with the type of the microorganisms.

Examples of the pH condition include pH 6 to 9, with pH 7 to 8.5 being more preferred.

Examples of the temperature condition include 20 to 40°C, with 25 to 37°C being preferred.

The culture is preferably shaking culture or aerated culture.

The culture time is not limited to a particular time and can be set as appropriate in accordance with the type of the microorganism and the amount of the culture medium. Examples of the culture time include 15 to 50 hours and 20 to 40 hours.

After the culture, bacterial cells are removed from the culture liquid by centrifugation, filtration, or the like, and the culture supernatant is collected. In addition to aspartic acid produced by the microorganisms, the culture supernatant contains various substances such as culture medium components and by-products by the microorganisms. Thus, processing to purify aspartic acid from the culture supernatant may be performed using a method commonly used in separation and purification processing such as concentration, isoelectric crystallization, recrystallization, activated carbon processing, or washing. By making the content of the impurity 0.02% by mass or more and 1.1% by mass or less by the purification processing, the aspartic acid composition can be obtained. The aspartic acid composition obtained by purifying the culture supernatant of the microorganisms usually contains, as the impurity, at least one selected from the group consisting of an amino acid other than aspartic acid and an organic acid.

When the aspartic acid composition is obtained by the method of reacting fumaric acid and ammonia in the presence of an enzyme catalyst to produce aspartic acid, the reaction solution after the reaction is collected, and aspartic acid purification processing is performed. By making the content of the impurity 0.02% by mass or more and 1.1% by mass or less by the purification processing, the aspartic acid composition can be obtained.

When the content of the impurity in the composition becomes less than 0.02% by mass by the aspartic acid purification processing, at least one component selected from the group consisting of an amino acid other than aspartic acid, an organic acid, and salts thereof may be added, so that the content of the impurity becomes 0.02% by mass or more and 1.1% by mass or less. In this case, it is preferable to add the amino acid other than aspartic acid.

Due to problems such as the depletion of petroleum resources, it is preferable that petroleum-derived products be replaced by those produced utilizing microorganisms. The aspartic acid composition for use in the production of polysuccinimide is also preferably produced using microorganisms. However, the culture supernatant of the microorganisms contains many impurities apart from aspartic acid, and thus it is difficult to purify the aspartic acid composition to a purity equivalent to that of petroleum-derived products (for example, 99.9 % by mass or more).

On the other hand, the aspartic acid composition of the present aspect can produce polysuccinimide with a sufficiently high molecular weight at a lower purity than that of petroleum-derived products. Furthermore, the polymerization rate of aspartic acid is promoted by using the aspartic acid composition of the present aspect (refer to FIG. 1). Thus, the purification cost of aspartic acid can be reduced, and conversion to an efficient process using the microorganisms can be expected.

It is thought that when the aspartic acid composition of the present aspect is used, the polymerization environment improves by the presence of the impurity such as the amino acid or the organic acid in a certain amount, thus improving the polymerization rate of aspartic acid compared to petroleum refined products, which have a higher purity of aspartic acid.

[Method for Producing Polysuccinimide]

In one aspect, the present invention provides a method for producing polysuccinimide, the method including performing a polymerization reaction of aspartic acid using the aspartic acid composition.

The polymerization reaction of aspartic acid can be performed by known methods. The polymerization reaction of aspartic acid may be performed under an acid catalyst or performed without catalyst. Examples of the acid catalyst include phosphoric acid, phosphorous acid, phosphites, and p-toluenesulfonic acid. The use amount of the acid catalyst is not limited to a particular amount, and examples thereof include 0.001 to 500 parts by mass with respect to 100 parts by mass of the aspartic acid composition, with 0.01 to 100 parts by mass being preferred and 0.1 to 70 parts by mass being more preferred.

The polymerization reaction of aspartic acid may be performed by solid phase polymerization or performed by solution polymerization.

(Solid Phase Polymerization)

The solid phase polymerization is a method of polymerization not using any reaction solvents. When the polymerization reaction is performed under a catalyst, the catalyst is preferably added to the aspartic acid composition and thoroughly mixed therewith before performing the polymerization reaction. The mixing temperature is not limited to a particular temperature so long as it is 150°C or lower, in which no polymerization reaction occurs. Examples of the mixing temperature include 20 to 120°C. Examples of the mixing time include 0.01 to 50 minutes.

The solid phase polymerization can be performed using a kneading apparatus after adding the catalyst to the aspartic acid composition. The catalyst may be added all at once or added separately in small amounts.

Although the kneading apparatus is not limited to a particular apparatus, an apparatus equipped with a stirring blade is preferred so as to facilitate steps after the polymerization. The stirring blade can perform stirring while crushing the polymer during the polymerization reaction. Examples of the type of the stirring blade include a screw blade, a helical ribbon blade, a horizontal biaxial blade, and a Bunbury blade, among which the screw blade is preferred. Examples of the kneading apparatus include kneaders, extruders, planetary mixers, helical blade mixers, and screw type mixers. The kneading apparatus may be of the batch type or the continuous type.

The polymerization reaction can be performed by charging the aspartic acid composition or a mixture of the aspartic acid composition and the acid catalyst into a reactor and heating it. Examples of the polymerization temperature include 100 to 400°C, with 150 to 350°C being preferred and 150 to 280°C being more preferred.

The pressure during the polymerization reaction is not limited to a particular pressure, and the polymerization reaction may be performed under negative pressure or the polymerization reaction may be performed under normal pressure. Examples of the pressure include 1 to 100 kPa.

The polymerization time is not limited to a particular time and may be a time sufficient to produce polysuccinimide with a desired length. Examples of the polymerization time include 0.5 to 100 hours, with 1 to 50 hours being preferred and 1 to 20 hours being more preferred.

The polymerization reaction may be performed under an inert gas flow such as nitrogen or argon. In addition, various additives such as chain-length extenders and reaction accelerators may also be used.

After the polymerization reaction, the reaction mixture may be pulverized and washed using a solvent such as methanol or washed using distilled water.

After washing, powder of polysuccinimide can be obtained by drying. Examples of the method of drying include natural drying, heat drying, and vacuum drying.

(Solution Polymerization)

The solution polymerization is a method of polymerization using a reaction solvent. Examples of the reaction solvent include aromatic hydrocarbons, halogenated hydrocarbons, ethers, esters, and aprotic polar solvents. The reaction solvent is a solvent with a boiling point of 100°C or higher, and a solvent with a boiling point of 130°C or higher is preferred. Examples of the aromatic hydrocarbons include cumene and mesitylene. Examples of the halogenated hydrocarbons include chlorotoluene and chlorobenzene. Examples of the ethers include anisole. Examples of the esters include n-amyl acetate. Examples of the aprotic polar solvents include N,N-dimethylformamide, N,N-dimethylacetamide, N-methylpyrrolidone, 1,3-dimethyl-2-imidazolidinone, tetramethylurea, dimethyl sulfoxide, sulfolane, and hexamethylphosphoramide. The reaction solvent may be used alone or may be a mixed solvent of two or more.

Among them, because of having a moderate boiling point, the reaction solvent is preferably cumene, chlorotoluene, xylene, mesitylene, pseudocumene, 1,3-dimethyl-2-imidazolidinone, sulfolane, or mixed solvents of two or more of these solvents.

The use amount of the reaction solvent is not limited to a particular amount, and examples thereof include 1 to 5,000 parts by mass with respect to 100 parts by mass of the aspartic acid composition, with 5 to 1,000 parts by mass being preferred and 10 to 500 parts by mass being more preferred.

The polymerization reaction can be performed by charging a mixture of the aspartic acid composition, an acid catalyst, and the reaction solvent into a reactor and heating the mixture while removing water being generated. Examples of the polymerization temperature include 100 to 400°C, with 120 to 350°C being preferred and 120 to 280°C being more preferred.

The pressure during the polymerization reaction is not limited to a particular pressure, and the polymerization reaction may be performed under negative pressure or the polymerization reaction may be performed under normal pressure.

The polymerization time is not limited to a particular time and may be a time sufficient to produce polysuccinimide with a desired length. Examples of the polymerization time include 0.5 to 100 hours, with 1 to 75 hours being preferred and 1 to 50 hours being more preferred.

The polymerization reaction may be performed under an inert gas flow such as nitrogen or argon. In addition, various additives such as chain-length extenders and reaction accelerators may also be used.

After the polymerization reaction, the reaction product may be washed using a solvent such as methanol and then washed using distilled water.

After washing, powder of polysuccinimide can be obtained by drying. Examples of the method of drying include natural drying, heat drying, and vacuum drying.

The weight average molecular weight (Mw) of polysuccinimide obtained by the polymerization reaction of the aspartic acid composition is not limited to a particular value. The polymerization reaction can be terminated when polysuccinimide with a desired weight average molecular weight is produced. Examples of the Mw of polysuccinimide include 50,000 or more and 60,000 or more. Examples of the range of the Mw of polysuccinimide include 10,000 to 200,000 and 20,000 to 200,000. Mw is a value in terms of polystyrene by the GPC method.

In the method of the present aspect, the polymerization reaction of aspartic acid is performed using the aspartic acid composition. Thus, polysuccinimide with a high molecular weight can be obtained. In addition, the polymerization reaction of aspartic acid is promoted, and thus the polymerization reaction time can be shortened.

[Polysuccinimide Composition]

In one aspect, the present invention provides a polysuccinimide composition. The polysuccinimide composition contains polysuccinimide and an impurity. The impurity contains at least one selected from the group consisting of an amino acid other than aspartic acid, an organic acid, and salts thereof. The content of the impurity in the polysuccinimide composition is 0.001% by mass or more and 1.1% by mass or less.

The "polysuccinimide composition" refers to a composition containing polysuccinimide as a main component. The polysuccinimide composition can be obtained by a polymerization reaction of the aspartic acid composition. For example, the polysuccinimide composition can be obtained by performing the method for producing polysuccinimide. The polysuccinimide composition can also be said to be a polymerization reaction product of the aspartic acid composition.

The polysuccinimide composition contains polysuccinimide and an impurity. The "impurity" in the polysuccinimide composition refers to components other than polysuccinimide contained in the polysuccinimide composition. In one embodiment, the impurity contains at least one selected from the group consisting of an amino acid other than aspartic acid and an organic acid. The impurity preferably contains the amino acid other than aspartic acid.

Examples of the amino acid as the impurity include the same as the amino acid as the impurity in the aspartic acid composition. The content of the amino acid as the impurity is preferably 0 part by mass or more and to 1.1 parts by mass or less or may be 0.001 part by mass or more and 1.1 parts by mass or less, 0.001 part by mass or more and 0.5 part by mass or less, or 0.001 part by mass or more and 0.3 part by mass or less with respect to 100 parts by mass of polysuccinimide.

The amino acid as the impurity may be in salt form. Examples of the salt of the amino acid include the same as above. The content ranges exemplified for the amino acid as the impurity also apply when the amino acid as the impurity is in salt form. When both the amino acid and the salt of the amino acid are contained as the impurity, the content ranges exemplified above apply to the total content of both.

Examples of the organic acid as the impurity include the same as the organic acid as the impurity in the aspartic acid composition. The content of the organic acid as the impurity is preferably 0 part by mass or more and 1.1 parts by mass or less, more preferably 0.001 part by mass or more and 0.5 part by mass or less, and even more preferably 0.001 part by mass or more and 0.3 part by mass or less with respect to 100 parts by mass of polysuccinimide.

The organic acid as the impurity may be in salt form. Examples of the salt of the organic acid include the same as above. The content ranges exemplified for the organic acid as the impurity also apply when the organic acid as the impurity is in salt form. When both the organic acid and the salt of the organic acid are contained as the impurity, the content ranges exemplified above apply to the total content of both.

The content of the impurity being within the above range promotes a hydrolysis reaction and/or a cross-linking reaction of polysuccinimide without problems. Consequently, a desired cross-linked polyaspartic acid composition can be obtained.

The Mw of polysuccinimide that the polysuccinimide composition contains as a main component is not limited to a particular value. Examples of the Mw of polysuccinimide include 50,000 or more and 60,000 or more. Examples of the range of the Mw of polysuccinimide include 50,000 to 200,000 and 60,000 to 200,000.

The impurity may contain components other than the amino acid other than aspartic acid, the organic acid, and salts thereof. Examples of the components include sulfates.

The content of polysuccinimide in the polysuccinimide composition is, for example, 99% by mass or more and 99.999% by mass or less with respect to the total mass of all the components of the polysuccinimide composition.

The polysuccinimide composition can be used for production of polyaspartic acid.

[Polyaspartic Acid Composition and Method for Producing Polyaspartic Acid Composition]

In one aspect, the present invention provides a polyaspartic acid composition, which is a hydrolyzed product of the polysuccinimide composition.

Hydrolyzing the polysuccinimide composition hydrolyzes polysuccinimide in the polysuccinimide composition to be polyaspartic acid. Thus, the polyaspartic acid composition can be obtained as a hydrolyzed product of the polysuccinimide composition. In the polyaspartic acid composition, polyaspartic acid is usually present in salt form. In the present specification, the term "polyaspartic acid" also encompasses a salt of polyaspartic acid unless otherwise expressly indicated.

The polyaspartic acid composition is a hydrolyzed product of the polysuccinimide composition and may thus contain the same impurity as in the polysuccinimide composition.

Examples of the amino acid as the impurity include the same as the amino acid as the impurity in the aspartic acid composition. The content of the amino acid as the impurity may be 0 part by mass or more and 1.1 parts by mass or less, 0.02 part by mass or more and 1.1 parts by mass or less, 0.02 part by mass or more and 1.0 part by mass or less, 0.001 part by mass or more and 0.98 part by mass or less, 0.005 part by mass or more and 0.95 part by mass or less, 0.010 part by mass or more and 0.90 part by mass or less, 0.020 part by mass or more and 0.80 part by mass or less, 0.05 part by mass or more and 0.70 part by mass or less, 0.05 part by mass or more and 0.50 part by mass or less, or 0.05 part by mass or more and 0.30 part by mass or less with respect to 100 parts by mass of polyaspartic acid.

These values of the upper limit and the lower limit are used in any combination.

The amino acid as the impurity may be in salt form. Examples of the salt of the amino acid include the same as above. The content ranges exemplified for the amino acid as the impurity also apply when the amino acid as the impurity is in salt form. When both the amino acid and the salt of the amino acid are contained as the impurity, the content ranges exemplified above apply to the total content of both.

The content of the organic acid as the impurity may be 0 part by mass or more and 1.1 parts by mass or less, 0.001 part by mass or more and 1.1 parts by mass or less, 0.02 part by mass or more and 1.1 parts by mass or less, 0.02 part by mass or more and 1.0 part by mass or less, 0.001 part by mass or more and 0.98 part by mass or less, 0.005 part by mass or more and 0.95 part by mass or less, 0.010 part by mass or more and 0.90 part by mass or less, 0.020 part by mass or more and 0.80 part by mass or less, 0.05 part by mass or more and 0.70 part by mass or less, 0.05 part by mass or more and 0.50 part by mass or less, or 0.05 part by mass or more and 0.30 part by mass or less with respect to 100 parts by mass of polyaspartic acid. These values of the upper limit and the lower limit are used in any combination.

The organic acid as the impurity may be in salt form. Examples of the salt of the organic acid include the same as above. The content ranges exemplified for the organic acid as the impurity also apply when the organic acid as the impurity is in salt form. When both the organic acid and the salt of the organic acid are contained as the impurity, the content ranges exemplified above apply to the total content of both.

The polyaspartic acid composition contains the impurity in an amount of 0.02% by mass or more 1.1% by mass or less with respect to the total mass of all the components of the polyaspartic acid composition. The content of the impurity may be 0.021% by mass or more and 1.1% by mass or less, 0.025% by mass or more and 1.1% by mass or less, 0.02% by mass or more and 1.0% by mass or less, 0.02% by mass or more and 0.98% by mass or less, 0.02% by mass or more and 0.95% by mass or less, 0.02% by mass or more and 0.90% by mass or less, 0.020% by mass or more and 0.80% by mass or less, 0.02% by mass or more and 0.70% by mass or less, 0.05% by mass or more and 0.50% by mass or less, or 0.05% by mass or more and 0.30% by mass or less with respect to the total mass of all the components of the polyaspartic acid composition. These values of the upper limit and the lower limit are used in any combination.

Thus, in one aspect, the present invention provides a method for producing a polyaspartic acid composition, the method including a step of performing a polymerization reaction of the aspartic acid using the aspartic acid composition to obtain a polysuccinimide composition containing polysuccinimide (hereinafter, also referred to as "Step (i)") and a step of performing a hydrolysis reaction of the polysuccinimide using the polysuccinimide composition to obtain a polyaspartic acid composition containing polyaspartic acid (hereinafter also referred to as "Step (ii) ") .

<Step (i)>

Step (i) is a step of performing a polymerization reaction of the aspartic acid using the aspartic acid composition to obtain a polysuccinimide composition containing polysuccinimide. Step (i) can be performed in the same manner as the method described in the above section [Method for Producing Polysuccinimide] .

<Step (ii)>

Step (ii) is a step of performing a hydrolysis reaction of the polysuccinimide using the polysuccinimide composition to obtain a polyaspartic acid composition containing polyaspartic acid.

The hydrolysis reaction of the polysuccinimide in the polysuccinimide compositions can be performed by known methods. The hydrolysis reaction can be performed under an alkaline condition. For example, the hydrolysis reaction of the polysuccinimide can be performed by adding a base to the polysuccinimide composition to be reacted with the polysuccinimide.

Examples of the base include hydroxides of alkali metals, carbonates of alkali metals, hydroxides of alkaline earth metals, carbonates of alkaline earth metals, and ammonia. Examples of the alkali metals include potassium, sodium, and lithium. Examples of the alkaline earth metals include calcium and magnesium. Among them, hydroxides of the alkali metals are preferred, NaOH, KOH, LiOH, and the like are more preferred, and an aqueous solution of NaOH is even more preferred. The base is preferably added to the polysuccinimide composition as an aqueous alkaline solution. The aqueous alkaline solution has a pH at 25°C of preferably 10 to 14 and more preferably 12 to 14. The concentration of the base in the aqueous alkaline solution is not limited to a particular concentration, and examples thereof include 0.5 to 50% by mass.

The use amount of the base may be an amount sufficient to hydrolyze the polysuccinimide in the polysuccinimide composition to the polyaspartic acid. For example, the number of moles of the base can be within a range of 90 to 120% of the combined number of moles of carboxy groups and imide groups of the polysuccinimide in the polysuccinimide composition.

The temperature in the hydrolysis reaction may be any temperature at which hydrolysis of the polymer main chain does not occur. The temperature in the hydrolysis reaction is, for example, preferably 10 to 70°C and more preferably 40 to 60°C.

The time for the hydrolysis reaction is not limited to a particular time, may be a time sufficient to hydrolyze the polysuccinimide in the polysuccinimide composition to the polyaspartic acid, and can be selected as appropriate in accordance with the amount of the polysuccinimide composition. Examples of the time for the hydrolysis reaction include 1 minute to 100 hours, with 30 minutes to 50 hours being preferred and 1 hour to 10 hours being more preferred.

The polyaspartic acid in the polyaspartic acid composition obtained in Step (ii) is in salt form. When a hydroxide or carbonate of an alkali metal is used as the base, the polyaspartic acid is in the form of a salt with the alkali metal. When a hydroxide or carbonate of an alkaline earth metal is used as the base, the polyaspartic acid is in the form of a salt with the alkaline earth metal. When ammonia is used as the base, the polyaspartic acid is in the form of a salt with the ammonium. The salt of the polyaspartic acid may be neutralized with an acid as appropriate to be made into polyaspartic acid forming no salt.

The polyaspartic acid composition may contain a polymer with a polysuccinimide structural unit partially remaining. For example, the polyaspartic acid composition may contain a polymer having an aspartic acid structural unit and a polysuccinimide structural unit. The ratio of the polysuccinimide structural unit in the polymer is preferably 3 mol% or less and more preferably 1 mol% or less with respect to all the structural units of the polymer.

[Cross-Linked Polyaspartic Acid Composition and Method for Producing Cross-Linked Polyaspartic Acid Composition]

In one aspect, the present invention provides a cross-linked polyaspartic acid composition, which is a cross-linking reaction product of the polyaspartic acid composition.

By performing a cross-linking reaction of the polyaspartic acid composition, the polyaspartic acid in the polyaspartic acid composition is cross-linked to be made into cross-linked polyaspartic acid. Thus, the cross-linked polyaspartic acid composition can be obtained as a cross-linking reaction product of the polyaspartic acid composition. In the cross-linked polyaspartic acid composition, the cross-linked polyaspartic acid may be in salt form or not. In the present specification, the term "cross-linked polyaspartic acid" also encompasses a salt of the cross-linked polyaspartic acid.

The cross-linked polyaspartic acid composition is a cross-linking reaction product of the polyaspartic acid composition and may thus contain the same impurity as in the polyaspartic acid composition.

Thus, in one aspect, the present invention provides a method for producing a cross-linked polyaspartic acid composition, the method including a step of obtaining a polyaspartic acid composition by the method for producing a polyaspartic acid composition (hereinafter, also referred to as "Step (iii)") and a step of performing a cross-linking reaction of the polyaspartic acid using the polyaspartic acid composition to obtain a cross-linked polyaspartic acid composition containing cross-linked polyaspartic acid (hereinafter, also referred to as "Step (iv)").

<Step (iii)>

Step (iii) is a step of obtaining a polyaspartic acid composition by the method for producing a polyaspartic acid composition. Step (iii) can be performed in the same manner as the method described in the above section [Polyaspartic Acid Composition and Method for Producing Polyaspartic Acid Composition].

<Step (iv)>

Step (iv) is a step of performing a cross-linking reaction of the polyaspartic acid using the polyaspartic acid composition to obtain a cross-linked polyaspartic acid composition containing cross-linked polyaspartic acid.

The cross-linking reaction of the polyaspartic acid in the polyaspartic acid composition can be performed by known methods. The cross-linking reaction can be performed using a cross-linking agent. For example, the cross-linking reaction of the polyaspartic acid can be performed by adding the cross-linking agent to the polyaspartic acid composition to be reacted with the polyaspartic acid. Alternatively, the cross-linking reaction of the polyaspartic acid can be performed by irradiating the polyaspartic acid composition with γ-rays.

The cross-linking agent is not limited to a particular cross-linking agent, and any known cross-linking agents can be used. Examples of the cross-linking agent include polyvalent epoxy compounds and basic polyamino acid compounds.

Examples of the polyvalent epoxy compounds include polyglycidyl ethers of alkane polyols (with a carbon atom number of 2 to 6, for example) and poly(alkylene glycol) (with a carbon atom number of 2 to 6, for example) such as ethylene glycol diglycidyl ether, diethylene glycol diglycidyl ether, polyethylene glycol diglycidyl ether, glycerin diglycidyl ether, glycerin triglycidyl ether, diglycerol polyglycidyl ether, polyglycerol polyglycidyl ether, propylene glycol diglycidyl ether, and butanediol diglycidyl ether; polyglycidyl ethers of alkane polyols (with a carbon atom number of 2 to 6, for example) and poly(alkylene glycol) (with a carbon atom number of 2 to 6, for example) such as sorbitol polyglycidyl ether, pentaerythritol polyglycidyl ether, erythritol polyglycidyl ether, trimethylolethane polyglycidyl ether, and trimethylolpropane polyglycidyl ether; diepoxyalkanes (with a carbon atom number of 4 to 8, for example) such as 1,2,3,4-diepoxybutane, 1,2,4,5-diepoxypentane, 1,2,5,6-diepoxyhexane, 1,2,7,8-diepoxyoctane, and 1,4- and 1,3-divinylbenzene epoxide; and polyphenol polyglycidyl ethers (with a carbon atom number of 6 to 15, for example) such as 4,4'-isopropylidene diphenol diglycidyl ether (bisphenol A diglycidyl ether) and hydroquinone diglycidyl ether. The polyvalent epoxy compounds may be used alone, or two or more may be used in combination.

Examples of the basic polyamino acid compounds include dimers containing basic amino acids such as glycine-lysine, lysine-glycine, alanine-lysine, and lysine-phenylalanine; polylysine, polyarginine, copolymers of lysine and arginine, and copolymers of a basic amino acid and another amino acid. The basic polyamino acid compounds may form salts with inorganic acids, organic acids, or the like. The basic polyamino acid compounds may be used alone, or two or more may be used in combination.

The use amount of the cross-linking agent may be an amount sufficient for the polyaspartic acid in the polyaspartic acid composition to cross-link. The use amount of the cross-linking agent can be selected as appropriate in accordance with the degree to which the polyaspartic acid is cross-linked. The use amount of the cross-linking agent is not limited to a particular amount, and examples thereof include 0.5 to 50 parts by mass with respect to 100 parts by mass of the polyaspartic acid composition, with 1 to 20 parts by mass being preferred and 10 to 10 parts by mass being more preferred.

The temperature in the cross-linking reaction can be selected as appropriate in accordance with the type of the cross-linking agent. When the cross-linking agent is a polyvalent epoxy compound, the temperature in the cross-linking reaction is, for example, preferably 10 to 120°C and more preferably 20 to 60°C. When the cross-linking agent is a basic polyamino acid compound, the temperature in the cross-linking reaction is preferably 10 to 120°C and more preferably 30 to 80°C.

The time for the cross-linking reaction is not limited to a particular time, may be a time sufficient for the polyaspartic acid in the polyaspartic acid composition to cross-link, and can be selected as appropriate in accordance with the amount of the polyaspartic acid composition. Examples of the time for the cross-linking reaction include 1 minute to 100 hours. The time for the cross-linking reaction, which is not limited to a particular time, is, for example, preferably 1 minute to 50 hours and more preferably 1 minute to 10 hours.

The polyaspartic acid in the polyaspartic acid composition used in Step (iv) may be in salt form or not. If the polyaspartic acid composition in salt form is used in Step (iv), the cross-linked polyaspartic acid in the resulting cross-linked polyaspartic acid composition is in salt form. If the polyaspartic acid composition not in salt form is used in Step (iv), the cross-linked polyaspartic acid in the resulting cross-linked polyaspartic acid composition is not in salt form. When the cross-linked polyaspartic acid in the cross-linked polyaspartic acid composition is in salt form, it may be neutralized with an acid as appropriate to be made into cross-linked polyaspartic acid not in salt form.

[Cross-Linked Polysuccinimide Composition and Method for Producing Cross-Linked Polysuccinimide Composition]

In one aspect, the present invention provides a cross-linked polysuccinimide composition, which is a cross-linking reaction product of the polysuccinimide composition.

In one aspect, the present invention provides a cross-linked polyaspartic acid composition, which is a hydrolyzed product of the cross-linked polysuccinimide composition.

By performing a cross-linking reaction of the polysuccinimide composition, the polysuccinimide in the polysuccinimide composition is cross-linked to be cross-linked polysuccinimide. Thus, the cross-linked polysuccinimide composition can be obtained as a cross-linking reaction product of the polysuccinimide composition.

The cross-linked polysuccinimide composition is a cross-linking reaction product of the polysuccinimide composition and may thus contain the same impurity as in the polysuccinimide composition.

By performing a hydrolysis reaction of the cross-linked polysuccinimide composition, the cross-linked polysuccinimide in the cross-linked polysuccinimide composition is hydrolyzed to be cross-linked polyaspartic acid. Thus, the cross-linked polyaspartic acid composition can be obtained as a hydrolyzed product of the cross-linked polysuccinimide composition. In the cross-linked polyaspartic acid composition, the cross-linked polyaspartic acid is usually present in salt form. In the present specification, the term "cross-linked polyaspartic acid" also encompasses a salt of the cross-linked polyaspartic acid unless otherwise expressly indicated.

The cross-linked polyaspartic acid composition is a hydrolyzed product of the cross-linked polysuccinimide composition and may thus contain the same impurity as in the cross-linked polysuccinimide composition.

Thus, in one aspect, the present invention provides a method for producing a cross-linked polyaspartic acid composition, the method including a step of performing a polymerization reaction of the aspartic acid using the aspartic acid composition to obtain a polysuccinimide composition containing polysuccinimide (hereinafter, also referred to as "Step (I)"), a step of performing a cross-linking reaction of the polysuccinimide using the polysuccinimide composition to obtain a cross-linked polysuccinimide composition containing cross-linked polysuccinimide (hereinafter, also referred to as "Step (II)"), and a step of performing a hydrolysis reaction of the cross-linked polysuccinimide using the cross-linked polysuccinimide composition to obtain a cross-linked polyaspartic acid composition containing cross-linked polyaspartic acid (hereinafter, also referred to "Step (III) ") .

<Step (I)>

Step (I) is a step of performing a polymerization reaction of the aspartic acid using the aspartic acid composition to obtain a polysuccinimide composition containing polysuccinimide. Step (I) can be performed in the same manner as the method described in the above section [Method for Producing Polysuccinimide] .

<Step (II)>

Step (II) is a step of performing a cross-linking reaction of the polysuccinimide using the polysuccinimide composition to obtain a cross-linked polysuccinimide composition containing cross-linked polysuccinimide.
The cross-linking reaction of the polysuccinimide in the polysuccinimide composition can be performed by known methods. The cross-linking reaction can be performed using a cross-linking agent. For example, the cross-linking reaction of the polysuccinimide can be performed by adding the cross-linking agent to the polysuccinimide composition to be reacted with the polysuccinimide. Alternatively, the cross-linking reaction of the polysuccinimide can be performed by irradiating the polysuccinimide composition with γ-rays.
The cross-linking agent is not limited to a particular cross-linking agent, and any known cross-linking agents can be used. Examples of the cross-linking agent include amine compounds, hydrazine, and chitosan.
Examples of the amine compounds include organic compounds having two or more amino groups. Examples of the amino group include a primary amino group and a secondary amino group. The primary amino group is preferred as the amino group because of its high reactivity with polysuccinimide and ease of adjusting the degree of cross-linking. The amino group is preferably an amino group replacing a hydrogen atom of an aliphatic hydrocarbon group because of its high reactivity with polysuccinimide. Specific examples of the amino group include an alkylamino group, an aralkylamino group, and a benzylamino group.
Examples of the amine compounds include aliphatic polyamines such as ethylenediamine, propylenediamine, 1,4-butanediamine, pentamethylenediamine, hexamethylenediamine, heptamethylenediamine, octamethylenediamine, nonamethylenediamine, decamethylenediamine, undecamethylenediamine, dodecamethylenediamine, tetradecamethylenediamine, hexadecamethylenediamine, 1-amino-2,2-bis(aminomethyl)butane, tetraaminomethane, diethylenetriamine, and triethylenetetramine; alicyclic polyamines such as norbornenediamine, 1,4-diaminocyclohexane, 1,3,5-triaminocyclohexane, and isophoronediamine; aromatic polyamines such as phenylenediamine, tolylenediamine, and xylylenediamine; polyamines such as basic amino acids and esters thereof; and polyamines such as compounds (for example, cystamine) in which one or more molecules of a monoamino compound are bonded together with one or more disulfide bonds and derivatives thereof. The amine compounds may also be amino acids having two or more amino groups, such as lysine, cystine, and ornithine, salts thereof, esters thereof, or the like.
Furthermore, specific examples of tri- or more functional amine compounds include 1,1,1-tris(2'-aminomethyl)ethane, tetrakis(2'-aminomethyl)methane, 1,1,1-tris(2'-aminoethyl)ethane, tetrakis(2'-aminoethyl)methane, 1,1,1-tris(2'-aminopropyl)ethane, tetrakis(2'-aminopropyl)methane, 1,1,1-tris(2'-aminobutyl)ethane, tetrakis(2'-aminobutyl)methane, 1,2,3-tris(aminomethyl)benzene, 1,2,4-tris(aminomethyl)benzene, and 1,3,5-tris(aminomethyl)benzene.
The cross-linking agent may be polymers of the amine compounds. Specific examples of the cross-linking agent as a polymer include dipeptides having a basic amino acid at the C-terminus, such as glycine-lysine, alanine-lysine, glycine-ornithine, and alanine-ornithine; dipeptides having a basic amino acid at the N-terminal, such as lysine-glycine, lysine-alanine, ornithine-glycine, ornithine-alanine, lysine-lysine, ornithine-lysine, lysine-ornithine, and ornithine-ornithine; oligopeptides containing at least one basic amino acid, such as glycine-glycine-lysine, glycine-lysine-glycine, glycine-glycine-ornithine, glycine-ornithine-glycine, lysine-lysine-lysine, ornithine-ornithine-ornithine, glycine-glycine-glycine-lysine, and glycine-ornithine-glycine-glycine; polypeptides such as polylysine, polyarginine, copolymers of lysine and arginine, copolymers of lysine and aspartic acid, copolymers of lysine and glutamic acid, copolymers of a basic amino acid and another amino acid; and polymers of amino group-containing monomers, such as polyethyleneimine, polyallylamine, chitosan, and peptides. When the cross-linking agent is a polymer, the degree of polymerization of the polymer is not limited to a particular one and can be selected as appropriate in consideration of solubility in an organic solvent used for the reaction and the like.
Examples of the cross-linking agent with high biodegradability and excellent reactivity with polysuccinimide include ethylenediamine, propylenediamine, 1,4-butanediamine, heptamethylenediamine, hexamethylenediamine, lysine, ornithine, and cystamine.

The use amount of the cross-linking agent may be an amount sufficient for the polysuccinimide in the polysuccinimide composition to cross-link. The use amount of the cross-linking agent can be selected as appropriate in accordance with the degree to which the polysuccinimide is cross-linked. The use amount of the cross-linking agent is not limited to a particular amount, and examples thereof include 0.5 to 50 parts by mass with respect to 100 parts by mass of the polysuccinimide composition, with 1 to 20 parts by mass being preferred and 10 to 10 parts by mass being more preferred.

The temperature in the cross-linking reaction can be selected as appropriate in accordance with the type of the cross-linking agent. The temperature in the cross-linking reaction is, for example, preferably 10 to 120°C and more preferably 20 to 60°C.

The time for the cross-linking reaction is not limited to a particular time, may be a time sufficient for the polysuccinimide in the polysuccinimide composition to cross-link, and can be selected as appropriate in accordance with the amount of the polysuccinimide composition. Examples of the time for the cross-linking reaction include 1 minute to 100 hours. The time for the cross-linking reaction, which is not limited to a particular time, is, for example, preferably 1 minute to 50 hours and more preferably 1 minute to 10 hours.

The cross-linking reaction may be performed in an organic solvent or performed in an aqueous solution. Examples of the solvent used for the cross-linking reaction include water; alcohols such as methanol, ethanol, propanol, isopropanol (2-propanol), butanol, pentanol, hexanol, heptanol, octanol, 2-methoxyethanol, and 2-ethoxyethanol; glycols such as ethylene glycol, diethylene glycol, triethylene glycol, propylene glycol, and dipropylene glycol; glycosolves such as ethylene glycol monomethyl ether and ethylene glycol monoethyl ether; ketones such as acetone, methyl ethyl ketone, and methyl isobutyl ketone; cyclic ethers such as tetrahydrofuran and dioxane; aliphatic hydrocarbons such as petroleum ether, pentane, hexane, heptane, octane, cyclohexane, and decalin; aromatic hydrocarbons such as benzene, toluene, ethylbenzene, and xylene; aromatic ethers such as diphenyl ether and anisole; and phenols such as cresol.

Furthermore, examples of the solvent used for the cross-linking reaction include N,N-dimethylformamide, N,N-dimethylacetamide, N-methylpyrrolidone, N,N'-dimethylimidazolidinone, dimethyl sulfoxide, and sulfolane. These solvents may be used alone, or two or more mixed together may be used.

<Step (III)>

Step (III) is a step of performing a hydrolysis reaction of the cross-linked polysuccinimide using the cross-linked polysuccinimide composition to obtain a cross-linked polyaspartic acid composition containing cross-linked polyaspartic acid.

The hydrolysis reaction of the cross-linked polysuccinimide in the cross-linked polysuccinimide composition can be performed by known methods. The hydrolysis reaction can be performed under an alkaline condition. For example, the hydrolysis reaction of the cross-linked polysuccinimide can be performed by adding a base to the cross-linked polysuccinimide composition to be reacted with the cross-linked polysuccinimide.

Examples of the base include hydroxides of alkali metals, carbonates of alkali metals, hydroxides of alkaline earth metals, carbonates of alkaline earth metals, ammonia, and organic amine compounds (triethylamine, N-methylmorpholine, triethanolamine, diisopropylethylamine, and the like). Examples of the alkali metals and alkaline earth metals include the same as above.

The use amount of the base may be an amount sufficient to hydrolyze the cross-linked polysuccinimide in the cross-linked polysuccinimide composition to the cross-linked polyaspartic acid. For example, the number of moles of the base can be within a range of 90 to 120% of the combined number of moles of carboxy groups and imide groups of the cross-linked polysuccinimide in the cross-linked polysuccinimide composition.

The temperature in the hydrolysis reaction may be any temperature at which hydrolysis of the polymer main chain does not occur. The temperature in the hydrolysis reaction is, for example, preferably 10 to 70°C and more preferably 40 to 60°C.

The time for the hydrolysis reaction is not limited to a particular time, may be a time sufficient to hydrolyze the cross-linked polysuccinimide in the cross-linked polysuccinimide composition to the cross-linked polyaspartic acid, and can be selected as appropriate in accordance with the amount of the cross-linked polysuccinimide composition. Examples of the time for the hydrolysis reaction include 1 minute to 100 hours. The time for the hydrolysis reaction is preferably 30 minutes to 50 hours and more preferably 1 hour to 10 hours.

The cross-linked polyaspartic acid in the cross-linked polyaspartic acid composition obtained in Step (III) is in salt form. When a hydroxide or carbonate of an alkali metal is used as the base, the cross-linked polyaspartic acid is in the form of a salt with the alkali metal. When a hydroxide or carbonate of an alkaline earth metal is used as the base, the cross-linked polyaspartic acid is in the form of a salt with the alkaline earth metal. When ammonia is used as the base, the cross-linked polyaspartic acid is in the form of a salt with the ammonium. When an organic amine is used as the base, the cross-linked polyaspartic acid is in the form of a salt with the organic amine. The salt of the cross-linked polyaspartic acid may be neutralized with an acid as appropriate to be made into cross-linked polyaspartic acid forming no salt.

The cross-linked polyaspartic acid composition may contain a polymer with a succinimide structural unit partially remaining. For example, the cross-linked polyaspartic acid composition may contain a polymer having an aspartic acid structural unit and a polysuccinimide structural unit. The ratio of the succinimide structural unit in the polymer is preferably 3 mol% or less and more preferably 1 mol% or less with respect to all the structural units of the polymer.

[Surface-Modified Cross-Linked Polyaspartic Acid Composition]

In one aspect, the present invention provides a surface-modified cross-linked polyaspartic acid composition. The surface-modified cross-linked polyaspartic acid composition contains particles of the cross-linked polyaspartic acid composition. The surfaces of the particles are modified with inorganic particles.

The cross-linked polyaspartic acid composition may be particulate, and its particle surface may be modified with inorganic particles. The inorganic particles are preferably water-insoluble inorganic particles. Examples of the inorganic particles include metal oxides such as aluminum oxide, titanium oxide, zinc oxide, and silicon dioxide.

The particles of the cross-linked polyaspartic acid composition can be obtained by, for example, physically pulverizing the cross-linked polyaspartic acid composition that has been dried. The surface-modified cross-linked polyaspartic acid composition can be obtained by reacting inorganic particles such as fumed silica with the particles of the cross-linked polyaspartic acid composition. The reaction between the cross-linked polyaspartic acid composition particles and the inorganic particles can be performed by, for example, heating them up to 100 to 200°C. The reaction time is not limited to a particular time, and examples thereof include 10 to 60 minutes.

[Cross-Linked Polyaspartic Acid-Containing Product]

In one aspect, the present invention provides a cross-linked polyaspartic acid-containing product containing the cross-linked polyaspartic acid composition.

In one aspect, the present invention provides a method for producing a cross-linked polyaspartic acid-containing product, the method including a step of obtaining a cross-linked polyaspartic acid composition by the method for producing a cross-linked polyaspartic acid composition and a step of producing a cross-linked polyaspartic acid-containing product using the cross-linked polyaspartic acid composition.

The cross-linked polyaspartic acid composition obtained as described above can be used for various uses. The "cross-linked polyaspartic acid-containing product" refers to a product containing the cross-linked polyaspartic acid composition. The cross-linked polyaspartic acid-containing product is not limited to a particular product so long as it is a product containing the cross-linked polyaspartic acid composition. Examples of the cross-linked polyaspartic acid-containing product include, but are not limited to, surfactants, viscosity-increasing agents, viscosity-reducing agents, swelling agents, water absorbents, gelators, binders, film-forming agents, flocculants, adhesives, surface modifiers, and sustained-release agents.

Product examples when the surface activating action of the cross-linked polyaspartic acid composition is utilized include emulsifiers, emulsion breakers, dispersants, flocculants, and antistatic agents. Examples of the dispersants include pigment dispersants, dispersants for agrochemical granules, dispersants for pulverized coal, cement dispersants, concrete admixtures, clean dispersants for lubricating oils, flow point depressants, plastic coloring aids, and compatibility agents. Examples of the flocculants include polymer flocculants, water filterability and yield improvers, and sludge flocculants. Product examples when the surface activating action of the cross-linked polyaspartic acid composition is utilized also include leveling agents, dye retardants, foaming agents, foam boosting agents, foaming-and-foam-boosting agents, foam stabilizers, soap bubble solutions, defoaming agents, solubilizers, life extenders for cut flowers, and lubricants.

Product examples when the viscosity-increasing action of the cross-linked polyaspartic acid composition is utilized include stabilizers, poultices, polymers for quench oils, polymers for hydraulic fluids, polymers for crude oil production increase (for example, polymers used in mining and well drilling (for example, temporary sealing materials, dispersion liquids for oil drilling assistance, flowability control materials, and polymers in well processing fluids), and glue agents for textile printing. Product examples when the viscosity-reducing action of the cross-linked polyaspartic acid composition is utilized include plasticizers.

Product examples when the swelling action of the cross-linked polyaspartic acid composition is utilized include carriers for drugs, drug volatilizers, drug carriers, materials related to electric equipment such as water stop materials for communication cables, gaskets and packings, sealants for civil engineering, sealants for construction, mud loss prevention agents for the shield construction method, water swelling toys, sealants, and artificial snow.

Product examples when the water-absorbing action of the cross-linked polyaspartic acid composition is utilized include humectants, dehumidifying agents, humidity regulators, moisture absorbents, and water retention materials.

Furthermore, examples of products containing these products include feminine hygiene products, diapers, breastfeeding pads, incontinence pads, medical underpads, disposable rags, pet sheets, portable toilets, disposable body warmers, dressing materials for wound protection, blood absorbents for medical use, sweat absorbent fibers, wet tissues, deodorizing-and-dehumidifying sheets, food freshness preservatives, dehydrating agents in the field of food and the like, food packaging materials such as drip absorbent sheets, transportation materials such as moisture absorbent sheets for transporting fresh vegetables, construction materials for condensation prevention, inkjet recording paper, moistureproof coating agents for printed wiring boards, waterproof agents for concrete, dehydrating agents for gasoline, moisture remover for gasoline, dehydrating agents for oils, and moisture removers for oils. Product examples in the agricultural field include water retention materials, soil conditioners, floral foam (cut flower immobilizing materials), seedbeds for raising seedlings, hydroponic vegetation sheets, seed tapes, fluid seeding media, agricultural sheets for condensation prevention, and irrigation liquids.

Product examples when the gelling action of the cross-linked polyaspartic acid composition is utilized include deodorants, deodorizers, cold insulators, and oil-absorbing polymers.

Product examples when the binding action of the cross-linked polyaspartic acid composition is utilized include scale inhibitors, tartar deposition inhibitors, heavy metal elution inhibitors, heavy metal immobilizers, selenium treatment agents, epoxy resin hardeners, builders, resin strengthening agents, dye fixing agents, dyeing inhibitors for fibers, fabric processing agents, and deodorization and antibacterial finishing agents for washing.

Product examples when the film-forming action of the cross-linked polyaspartic acid composition is utilized include rust inhibitors, packaging materials (for example, packaging materials for food, pharmaceuticals, electronic materials, and the like), general industrial films, films for packaging materials, coating agents, paints, inks, inkjet inks, water-based inks, ballpoint pen inks, metallic luster inks, erasable inks, refills for writing instruments, tablet coating agents, seamless capsule bases for storing drugs in the inner space, polymers for floor polishes, polymers for paints, masking agents, plastic hard coating agents, polymers for photoresists, coating agents for electromagnetic shielding, and fire extinguishing agents.

Product examples when the flocculating or adhesive action of the cross-linked polyaspartic acid composition is utilized include cultured cell sheets, flocculants, binders for printing inks, water-soluble binders, binders for nonwoven fabrics, binders for plastic reinforced fibers, binders for electrophotographic toners, binders for magnetic tapes, binders for resin concrete, binders for casting sand, binders for fine ceramics, sealants (for example, sealants for electronic devices, optical fibers, and the like), and adhesives.

Product examples when the surface modifying action of the cross-linked polyaspartic acid composition is utilized include fiber treatment agents, noodle quality improvers, and primers.

Product examples when the sustained-release action of the cross-linked polyaspartic acid composition is utilized include sustained-release agents (for example, base materials for sustained-release of active ingredients of cosmetics, pharmaceuticals, agrochemicals, fertilizers, and the like).

In addition, products containing the above products as raw materials are applicable to cosmetics, fragrances and cosmetics, pharmaceuticals, quasi-drugs, pharmaceutical-related materials, medical supplies, health-related supplies, hygiene products, daily commodities, agricultural materials, food-related materials, civil engineering-related materials, construction-related materials, electric equipment-related materials, electronic equipment-related materials, writing-related materials, painting-related materials, printing-related materials, electronic printing-related materials, manufacturing process-related materials, analysis-related materials, well-related materials, dyeing-related materials, fabric processing-related materials, paper manufacturing-related materials, materials for environmental measures treatment, and binders.

The cross-linked polyaspartic acid-containing product can be manufactured by known methods corresponding to the product, using the cross-linked polyaspartic acid composition as a raw material.

[Examples]

The following describes the present invention by examples, but the present invention is not limited to the following examples.

[Evaluation of Polymerization Reaction using Aspartic Acid Composition]

<Preparation of Aspartic Acid Composition>

(Example 1, Example 2, and Comparative Example 1)

Corynebacterium glutamicum was cultured, and the culture supernatant was purified to obtain an aspartic acid compound of each example.

(Reference Example 1)

L-aspartic acid manufactured by YIXING QIANCHENG BIOENGINEERING was purchased and used as an aspartic acid composition.

<Composition Analysis of Aspartic Acid Composition>

The amino acid was analyzed with an amino acid analyzer. The organic acid was analyzed by high-performance liquid chromatography. Table 1 lists the composition of the aspartic acid composition of each example. The values in Table 1 are % by mass with respect to the total mass (100% by mass) of the aspartic acid composition. "0.00" indicates less than 0.005% by mass.

[Table 1]

| | | Example 1 | Example 2 | Comparative Example 1 | Reference Example 1 |
|---|---|---|---|---|---|
| Main component | Aspartic acid | 99.7 | 99.02 | 98.63 | 99.97 |
| Impurity | Impurity content | 0.30 | 0.98 | 1.37 | 0.03 |
| | Glutamic acid | 0.14 | 0.25 | 0.33 | 0.00 |
| | Alanine | 0.15 | 0.31 | 0.53 | 0.00 |
| Amino acid | Valine | 0.00 | 0.17 | 0.22 | 0.00 |
| | Amino acid total | 0.29 | 0.73 | 1.08 | 0.00 |
| | Pyruvic acid | 0.00 | 0.00 | 0.00 | 0.00 |
| | Succinic acid | 0.00 | 0.00 | 0.00 | 0.00 |
| Organic acid | Acetic acid | 0.00 | 0.00 | 0.00 | 0.00 |
| | Malic acid | 0.00 | 0.25 | 0.00 | 0.00 |
| | Fumaric acid | 0.00 | 0.00 | 0.11 | 0.01 |
| | Organic acid total | 0.00 | 0.25 | 0.11 | 0.01 |
| Total of amino acid + organic acid | | 0.29 | 0.98 | 1.19 | 0.01 |
| [% by mass] | | | | | |

<Production of Polysuccinimide by Solid Phase Polymerization (1)>

Using the aspartic acid composition of each example, polysuccinimide was synthesized by solid phase polymerization. Mixed together in a mortar were 160 parts by mass of the aspartic acid composition and 97 parts by mass of 85% phosphoric acid, which were transferred to a tray and were reacted at 190°C and 1.3 kPa for 6 hours. During the reaction, sampling was performed as appropriate to measure the weight average molecular weight (Mw) of the polysuccinimide.

The reaction mixture was pulverized and was then washed using distilled water until the filtrate became neutral. The composition after being washed was vacuum dried at 80°C to obtain a polysuccinimide composition.

<Production of Polysuccinimide by Solid Phase Polymerization (2a)>

Using the aspartic acid composition of each example, polysuccinimide was synthesized by solid phase polymerization. Mixed together in a mortar were 160 parts by mass of the aspartic acid composition and 83 parts by mass of 85% phosphoric acid, which were transferred to a tray and were reacted at 190°C and 1.3 kPa for 6 hours. During the reaction, sampling was performed as appropriate to measure the weight average molecular weight (Mw) of the polysuccinimide.

The reaction mixture was pulverized and was then washed using distilled water until the filtrate became neutral. The composition after being washed was vacuum dried at 80°C to obtain a polysuccinimide composition.

<Production of Polysuccinimide by Solid Phase Polymerization using Kneader (Kneading Machine (2b))>

Using the aspartic acid composition of each example, polysuccinimide was synthesized by solid phase polymerization using a kneader. Charged into the kneader having a Bunbury blade were 160 parts by mass of the aspartic acid composition and 97 parts by mass of 85% phosphoric acid, which were mixed together for 10 minutes. Subsequently, using a heater block included in the kneader, the mixture was stirred while being heated from room temperature to an internal temperature of 190°C. After the temperature reached 190°C, a decompressed state of 9.3 kPa was made, and the mixture was reacted for 3 hours. The reaction mixture was pulverized and was then washed using distilled water until the filtrate became neutral. The composition after being washed was vacuum dried at 80°C to obtain a polysuccinimide composition.

<Measurement of Weight Average Molecular Weight (Mw) of Polysuccinimide>

For the weight average molecular weight (Mw) of polysuccinimide, a value in terms of polystyrene by the GPC method (a differential refractometer) was determined. For the measurement, G1000HHR column, G4000HHR column, and GMHHR-H column (TSKgel (registered trademark), Tosoh Corporation) were used. Dimethylformamide containing 10 mM lithium bromide was used as the eluent.

<Results>

FIG. 1 illustrates the results. In the legend of FIG. 1, the description in the parentheses indicates the method for producing polysuccinimide by solid phase polymerization. (1) indicates that the above method of production (1) was used, and (2a) indicates that the above method of production (2a) was used. The aspartic acid compositions of Example 1 and Example 2, despite their higher impurity content than in Reference Example 1, produced a polymerization reaction progressed more rapidly and polysuccinimide with a higher Mw than in Reference Example 1.
On the other hand, Comparative Example 1 produced a slower polymerization rate and a lower Mw of polysuccinimide than in the examples and the reference example.

<Production of Polysuccinimide by Solution Polymerization>

(Example 3)

Charged into a 1 L flask equipped with a stirrer, a thermometer, a Dean-Stark trap, and a nitrogen introducing tube were 60 parts by mass of the aspartic acid composition obtained in Example 2, 140 parts by mass of mesitylene, 60 parts by mass of sulfolane, and 2.6 parts by mass of 85% phosphoric acid, which were made into a uniform slurry state while adjusting the stirring state. Under heating and refluxing with an oil bath, the reaction was continued while removing azeotropic water. After the end of the reaction, the contents were filtered out, were washed with methanol, and were further washed using distilled water until the filtrate became neutral. The composition after being washed was vacuum dried at 80°C to obtain a polysuccinimide composition with a weight average molecular weight of 49,000.

(Example 4)

A polysuccinimide composition was synthesized in the same manner as in Example 3 except that the reaction solvent was changed to 80 parts by mass of xylene and 100 parts by mass of sulfolane. Consequently, a polysuccinimide composition with a weight average molecular weight of 80,000 was obtained.

(Example 5)

A polysuccinimide composition was synthesized in the same manner as in Example 3 except that the reaction solvent was changed to 100 parts by mass of xylene and 100 parts by mass of sulfolane. Consequently, a polysuccinimide composition with a weight average molecular weight of 111,000 was obtained.

[Evaluation of Cross-Linked Aspartic Acid Composition]

<Production of Cross-Linked Polyaspartic Acid Composition>

(Example 6 to Example 13)

Using the aspartic acid composition of Example 2, a polysuccinimide composition was obtained by the method described in <Production of Polysuccinimide by Solid Phase Polymerization (2a)>. Using the polysuccinimide composition, a cross-linked aspartic acid composition was produced by the following method.

The polysuccinimide composition in an amount of 100 parts by mass was dispersed in 167 parts of ion-exchanged water. While the dispersion liquid was stirred, 86 parts by mass of a 48% aqueous sodium hydroxide solution was added dropwise thereto using a dropping funnel. After the end of the dropwise addition, stirring was continued until there was no object left unsolved to obtain a polyaspartic acid composition with a nonvolatile (NV) component of 40%. The obtained polyaspartic acid composition was an aqueous solution of sodium polyaspartate.

The pH of 150 g of the polyaspartic acid composition was adjusted. Ethylene glycol diglycidyl ether (EGDE; Denacol EX-810 manufactured by Nagase ChemteX Corporation) in an addition amount of each example listed in Table 2 was added to the polyaspartic acid composition and was reacted at 60°C. It was reacted until gelation was confirmed, then the gel was collected, was washed with water, and was freeze-dried to obtain a solid of a cross-linked polyaspartic acid composition of each example. In the obtained cross-linked polyaspartic acid composition, cross-linked polyaspartic acid was present in the form of a sodium salt.

<Production of Cross-Linked Polyaspartic Acid Composition>

(Examples 14 and 15)

Using the aspartic acid composition of Example 2, a polysuccinimide composition was obtained by the method described in <Production of Polysuccinimide by Solid Phase Polymerization using Kneader (Kneading Machine (2b))>. A cross-linked polyaspartic acid composition was produced from the polysuccinimide composition in the same manner as in Example 6 to Example 13 except that the polysuccinimide composition was used.

<Production of Cross-Linked Polyaspartic Acid Composition>

(Examples 16 to 21)

Using the aspartic acid composition of Example 2, a polysuccinimide composition was obtained by the method described in <Production of Polysuccinimide by Solid Phase Polymerization (2a)>. Using the polysuccinimide composition, a cross-linked polyaspartic acid composition was produced by the following method.

(Examples 16 and 17)

The polysuccinimide composition in an amount of 10 parts by mass was mixed with 40 parts by mass of dimethylformamide and was dissolved by stirring at 60°C for 5 hours. While the solution was heated at 40°C, a solution in which 0.24 part by mass of hexamethylenediamine (HDA) and 2.2 parts by mass of dimethylformamide had been mixed together was added dropwise thereto, and the mixture was reacted for 48 hours while being held at 40°C. The obtained gel was added to 500 parts by mass of ion-exchanged water, which was pulverized with a mixer. After being filtered out, the gel was washed with 150 parts by mass of methanol for 1 hour and was vacuum dried at 60°C to obtain 10.1 g of a cross-linked polysuccinimide composition (PCI-1). The degree of cross-linking in this case was 2.0 mol%.

The cross-linked polysuccinimide composition (PCI-1) obtained above in an amount of 3.0 parts by mass was dispersed in a mixed solvent of 20 parts by mass of methanol and 10 parts by mass of ion-exchanged water. A solution in which 2.4 parts by mass of 48% sodium hydroxide and 2.4 parts by mass of methanol had been mixed together was divided into 10 solutions, each of which was added to the dispersion liquid every 30 minutes, and then the mixture was reacted for 15 hours. The reaction solution was charged into 400 parts by mass of methanol, and the precipitate was filtered out and collected and was further washed with 100 parts by mass of methanol. The precipitate was vacuum dried at 60°C to obtain 3.0 g of a cross-linked polyaspartic acid composition (cross-linked sodium polyaspartate).

(Examples 18 and 19)

A cross-linked polysuccinimide composition (PCI-2) in an amount of 10.1 g was obtained in the same manner as in Examples 16 and 17 except that the use amounts of the hexamethylenediamine (HDA) and dimethylformamide were 0.36 part by mass and 3.2 parts by mass, respectively. The degree of cross-linking in this case was 3.0 mol%.

A cross-linked polyaspartic acid composition (cross-linked sodium polyaspartate) in an amount of 3.0 g was obtained in the same manner as in Examples 16 and 17 except that the cross-linked polysuccinimide composition (PCI-2) was used.

(Examples 20 and 21)

A cross-linked polyaspartic acid composition (cross-linked sodium polyaspartate) in an amount of 3.0 g was obtained in the same manner as in Examples 16 and 17 except that the use amounts of the hexamethylenediamine (HDA) and dimethylformamide were 0.48 part by mass and 4.3 parts by mass, respectively. The degree of cross-linking in this case was 4.0 mol%.

(Comparative Example 2)

Using the aspartic acid composition of Comparative Example 1, a polysuccinimide composition was obtained by the method described in <Production of Polysuccinimide by Solid Phase Polymerization (1)>. A polyaspartic acid composition was obtained in the same manner as in Examples 6 to 13 except that the polysuccinimide composition was used. The pH of 150 g of the obtained polyaspartic acid composition was adjusted, and EGDE was added in an addition amount listed in Table 2 and was reacted at 60°C. Gelation of the reaction solution could not be confirmed, and a solid of a cross-linked polyaspartic acid composition could not be obtained. This was thought to be because the polymer chains of the polyaspartic acid in the polyaspartic acid composition did not have a length sufficient to gel by cross-linking.

<Method of Surface Treatment for Cross-Linked Polyaspartic Acid Composition>

For the cross-linked aspartic acid compositions of Examples 7, 9, 11, 13, 15, 17, 19, and 21, surface treatment was performed in accordance with the following method.

To 100 parts by mass of the cross-linked polyaspartic acid composition, which had been pulverized in a mortar and passed through meshes to have a particle size of 150 to 710 $\mu$m, 0.5 part by mass of fumed silica (AEROSIL 200 manufactured by Nippon Aerosil Co., Ltd.) was added, and they were mixed together well. Next, the mixture was heated at 140°C for 20 minutes to obtain a solid of a surface-treated cross-linked polyaspartic acid composition.

<Evaluation of Water Absorbency of Cross-Linked Polyaspartic Acid Composition>

The evaluation of the water absorbency of the cross-linked polyaspartic acid composition was performed in accordance with the teabag method (JIS K-7223) using a physiological saline solution. A water absorption amount was calculated by the following expression.

Water absorption amount [g - water/g] = {(weight after water absorption) - (blank weight after water absorption) - (sample weight)}/(sample weight)

<Evaluation of Water Retainability of Cross-Linked Polyaspartic Acid Composition>

The evaluation of the water retainability of the cross-linked polyaspartic acid composition was performed by, after the evaluation of the water absorption amount by the teabag method, performing dehydration with a centrifugal dehydrator under conditions of 25°C and 150G $\times$ 2 minutes and measuring the weight of the teabag after dehydration. A water retention amount was calculated by the following expression.

Water retention amount [g - water/g] = {(weight after dehydration) - (blank weight after dehydration) - (sample weight)}/(sample weight)

<Evaluation of Liquid Permeability of Cross-linked Polyaspartic Acid Composition>

The evaluation of the liquid permeability of the cross-linked polyaspartic acid composition was performed with reference to the method described in Japanese Unexamined Patent Application Publication No. 2012-41439 and the like. Specifically, 0.32 g of the cross-linked polyaspartic acid composition was immersed in 150 mL of a physiological saline solution (NaCl: 0.9% by mass) for 30 minutes to prepare swollen gel particles. Next, the prepared swollen gel particles together with the physiological saline solution were transferred into a filtration cylindrical pipe having a wire mesh (opening: 106 μm, JIS Z8801-1: 2006) and an openable and closable cock (inner diameter: 5 mm, length: 10 cm) at the bottom of an upright cylinder (diameter (inner diameter): 25.4 mm, length 40 cm, having scale lines at the 40 ml position and the 60 ml position from the bottom), with the cock closed. Next, a circular wire mesh (opening: 150 μm, diameter: 25 mm) was placed on the swollen gel particles such that the wire mesh and the swollen gel particles were in contact with each other. The used circular wire mesh included a pressurizing shaft (weight: 22 g, length: 47 cm) joined perpendicularly to the wire mesh face. In addition, a weight {88.5 g} was placed on the pressurizing shaft and was left at rest for 1 minute. Subsequently, the cock was opened, and the time (T1; seconds) required for the liquid level in the filtration cylindrical pipe to shift from the 60-mL scale line to the 40-mL scale line was measured. The temperatures of the physiological saline solution and the measurement atmosphere were set at 25°C ± 2°C. The liquid permeability was calculated by the following expression.
$$\text{Liquid permeability [mL/min]} = 20 \text{ mL} \times 60/(T1 - T2)$$

T1: time (seconds) for 20 mL of the physiological saline solution to pass through a sample under measurement.
T2: time (seconds) for 20 mL of the physiological saline solution to pass without a sample under measurement.
Tables 2 and 3 summarize the evaluation results of the water absorption amount, the water retention amount, and the liquid permeability. In Tables 2 and 3, "gelation" indicates whether a gelated cross-linked polyaspartic acid composition was obtained (A: gelated; B: not gelated). In Table 2, "ND" indicates detection limit or less.

[Table 2]

| | | Example 6 | Example 7 | Example 8 | Example 9 | Example 10 | Example 11 | Example 12 | Example 13 | Example 14 | Example 15 | Comparative Example 2 |
|---|---|---|---|---|---|---|---|---|---|---|---|---|
| Use amount of polyaspartic acid (g) | | 150 | 150 | 150 | 150 | 150 | 150 | 150 | 150 | 150 | 150 | 150 |
| EGDE addition amount (g) | | 1.3 | 1.3 | 2 | 2 | 3 | 3 | 4 | 4 | 2 | 2 | 4 |
| Gelation | | A | A | A | A | A | A | A | A | A | A | B |
| Surface treatment | | Undone | Done | Undone | Done | Undone | Done | Undone | Done | Undone | Done | NA |
| Water absorbency evaluation results | Water absorption amount (g/g) | 44 | 67 | 40 | 43 | 42 | 40 | 41 | 41 | 39 | 42 | Not measurable |
| | Water retention amount (g/g) | 37 | 32 | 24 | 20 | 23 | 17 | 21 | 17 | 24 | 20 | Not measurable |
| | Liquid permeability (ml/min) | <1 | <1 | <1 | 170 | <1 | 340 | ND | 150 | <1 | 190 | Not measurable |

EP 4 365 219 A1

[Table 3]

|  |  | Example 16 | Example 17 | Example 18 | Example 19 | Example 20 | Example 21 |
|---|---|---|---|---|---|---|---|
| Use amount of polysuccinimide (g) |  | 10 | 10 | 10 | 10 | 10 | 10 |
| HDA addition amount (g) |  | 0.24 | 0.24 | 0.36 | 0.36 | 0.48 | 0.48 |
| Gelation |  | A | A | A | A | A | A |
| Surface treatment |  | Undone | Done | Undone | Done | Undone | Done |
| Water absorbenc y evaluatio n results | Water absorption amount (g/g) | 44 | 45 | 35 | 36 | 23 | 24 |
|  | Water retention amount (g/g) | 34 | 30 | 27 | 23 | 19 | 15 |
|  | Liquid permeabili ty (ml/min) | ND | <1 | <1 | 140 | <1 | 370 |

It has been confirmed from the results in Tables 2 and 3 that the cross-linked polyaspartic acid compositions of Examples 6 to 21 have excellent water absorbency and water retainability.

It has been confirmed that the surface-treated cross-linked polyaspartic acid compositions of Example 9, Example 11, Example 13, Example 15, Example 17, Example 19, and Example 21 show excellent liquid permeability. In the surface-treated cross-linked polyaspartic acid composition of Example 7, no improvement in the liquid permeability by the surface treatment was confirmed. This is thought to be because the amount of the cross-linking agent for obtaining the cross-linked polyaspartic acid composition was small, and thus sufficient cross-linking density was not obtained and only soft gels were formed. It is thought that the liquid permeability could not be ensured for that reason.

Although the preferred embodiments of the present invention have been described and illustrated above, it should be understood that these are illustrative of the present invention and should not be considered limiting. Additions, omissions, substitutions, and other changes can be made without departing from the spirit or scope of the present invention. Thus, the present invention is not considered limited by the foregoing description but is limited only by the scope of the appended claims.

Claims

1. An aspartic acid composition comprising aspartic acid and an impurity,

the impurity containing at least one selected from the group consisting of an amino acid other than aspartic acid, an organic acid, and salts of the amino acid other than aspartic acid and the organic acid,
a content of the impurity being 0.02% by mass or more and 1.1% by mass or less.

2. The aspartic acid composition according to claim 1, wherein the impurity contains the amino acid other than aspartic acid.

3. The aspartic acid composition according to claim 1 or 2, wherein the aspartic acid is produced by microorganisms.

4. A method for producing polysuccinimide, the method comprising performing a polymerization reaction of the aspartic acid using the aspartic acid composition according to any one of claims 1 to 3.

5. The method for producing polysuccinimide according to claim 4, wherein the polymerization reaction is performed within a kneading apparatus.

6. A polysuccinimide composition comprising polysuccinimide and an impurity,

the impurity containing at least one selected from the group consisting of an amino acid other than aspartic acid, an organic acid, and salts of the amino acid other than aspartic acid and the organic acid, a content of the impurity being 0.001% by mass or more and 1.1% by mass or less.

7. The polysuccinimide composition according to claim 6, wherein the impurity contains the amino acid other than aspartic acid.

8. A polysuccinimide composition, being a polymerization reaction product of the aspartic acid composition according to any one of claims 1 to 3.

9. A polyaspartic acid composition, being a hydrolyzed product of the polysuccinimide composition according to any one of claims 6 to 8.

10. A polyaspartic acid composition comprising polyaspartic acid and an impurity,

   the impurity containing at least one selected from the group consisting of an amino acid other than aspartic acid, an organic acid, and salts of the amino acid other than aspartic acid and the organic acid, a content of the impurity being 0.001% by mass or more and 1.1% by mass or less.

11. The polyaspartic acid composition according to claim 10, wherein the impurity contains the amino acid other than aspartic acid or a salt of the amino acid other than aspartic acid.

12. A cross-linked polyaspartic acid composition, being a cross-linking reaction product of the polyaspartic acid composition according to any one of claims 9 to 11.

13. A cross-linked polyaspartic acid composition, being a hydrolyzed product of a cross-linking reaction product of the polysuccinimide composition according to any one of claims 6 to 8.

14. A surface-modified cross-linked polyaspartic acid composition comprising particles of the cross-linked polyaspartic acid composition according to claim 12 or 13, surfaces of the particles being modified with inorganic particles.

15. A cross-linked polyaspartic acid-containing product comprising the cross-linked polyaspartic acid composition according to claim 12 or 13 or the surface-modified cross-linked polyaspartic acid composition according to claim 14.

16. The cross-linked polyaspartic acid-containing product according to claim 15, wherein the cross-linked polyaspartic acid-containing product is a surfactant, a viscosity-increasing agent, a viscosity-reducing agent, a swelling agent, a water absorbent, a gelator, a binder, a film-forming agent, a flocculant, an adhesive, a surface modifier, or a sustained-release agent.

## FIG. 1

**INTERNATIONAL SEARCH REPORT**

| International application No. |
| --- |
| **PCT/JP2022/016512** |

| | |
| --- | --- |
| **A.** | **CLASSIFICATION OF SUBJECT MATTER** |

*C08G 73/10*(2006.01)i; *C08J 3/12*(2006.01)i; *C08J 3/24*(2006.01)i
FI:   C08G73/10; C08J3/12 A CEZ; C08J3/24 Z CFG

According to International Patent Classification (IPC) or to both national classification and IPC

| | |
| --- | --- |
| **B.** | **FIELDS SEARCHED** |

Minimum documentation searched (classification system followed by classification symbols)

C08G73/10; C08J3/12; C08J3/24

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | JP 09-316196 A (MITSUBISHI CHEM CORP) 09 December 1997 (1997-12-09) | 1-11 |
| | claims 1-2, paragraphs [0001]-[0004], [0007], [0009], [0018]-[0026], examples 1-3 | |
| Y | | 12-13, 15-16 |
| A | | 14 |
| X | JP 63-233958 A (AJINOMOTO CO INC) 29 September 1988 (1988-09-29) | 1-3 |
| | claim 1, p. 1, lower left column, line 15 to p. 1, lower right column, line 9, p. 2, upper right column, line 4 to p. 2, lower right column, line 6, example 2 | |
| A | | 4-16 |
| X | JP 2000-166591 A (NIPPON SHOKUBAI CO LTD) 20 June 2000 (2000-06-20) | 1-3 |
| | claim 1, paragraphs [0025], [0043]-[0044], [0050]-[0099] | |
| A | | 4-16 |
| Y | JP 10-324750 A (MITSUI CHEM INC) 08 December 1998 (1998-12-08) | 12-13, 15-16 |
| | paragraphs [0025]-[0026], [0034] | |
| A | JP 08-143515 A (SUMITOMO CHEM CO LTD) 04 June 1996 (1996-06-04) | 1-16 |
| | paragraph [0007] | |

☑ Further documents are listed in the continuation of Box C.     ☑ See patent family annex.

| | |
| --- | --- |
| *     Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **16 June 2022** | **28 June 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
|---|
| **PCT/JP2022/016512** |

### C. DOCUMENTS CONSIDERED TO BE RELEVANT

| Category* | Citation of document, with indication, where appropriate, of the relevant passages | Relevant to claim No. |
|---|---|---|
| A | JP 2018-150226 A (AGC SI TECH CO LTD) 27 September 2018 (2018-09-27)<br>claim 1 | 1-16 |

Form PCT/ISA/210 (second sheet) (January 2015)

**INTERNATIONAL SEARCH REPORT**
Information on patent family members

International application No.

**PCT/JP2022/016512**

| Patent document cited in search report | | | Publication date (day/month/year) | Patent family member(s) | | | Publication date (day/month/year) |
|---|---|---|---|---|---|---|---|
| JP | 09-316196 | A | 09 December 1997 | (Family: none) | | | |
| JP | 63-233958 | A | 29 September 1988 | US | 4922011 | A | |
| | | | | claim 1, column 1, lines 10-41, column 3, lines 1-15, example 2 | | | |
| JP | 2000-166591 | A | 20 June 2000 | EP | 994189 | A1 | |
| | | | | claim 1, paragraphs [0030], [0050]-[0052], [0086]-[0124] | | | |
| | | | | US | 6821760 | B1 | |
| JP | 10-324750 | A | 08 December 1998 | US | 6072024 | A | |
| | | | | column 3, line 14 to column 6, line 32 | | | |
| | | | | US | 6346569 | B1 | |
| | | | | EP | 866084 | A2 | |
| | | | | CN | 1198444 | A | |
| | | | | CN | 1422886 | A | |
| JP | 08-143515 | A | 04 June 1996 | KR | 10-0433748 | B1 | |
| | | | | CN | 1131146 | A | |
| | | | | CN | 1526696 | A | |
| | | | | CN | 1524845 | A | |
| | | | | CN | 1680273 | A | |
| JP | 2018-150226 | A | 27 September 2018 | (Family: none) | | | |

Form PCT/ISA/210 (patent family annex) (January 2015)

## REFERENCES CITED IN THE DESCRIPTION

### Patent documents cited in the description

- JP 2021107638 A
- JP H09176312 A
- WO 2020208842 A
- JP 2012041439 A

### Non-patent literature cited in the description

- Bargeys Manual of Determinative Bacteriology. 1974, vol. 8, 599